# EUROPEAN PATENT SPECIFICATION

(11) **EP 4 709 431 B1**
(45) Date of publication and mention of the grant of the patent: **23.09.2026**
(21) Application number: 24739078.4
(22) Date of filing: 13.05.2024
(51) Int. Cl.: A61L 2/20, A61L 11/00

(54) **DEVICE FOR DISINFECTION OF MATERIAL WITH OZONE**
GERÄT ZUR DESINFEKTION VON MATERIAL MIT OZON
DISPOSITIF DE DÉSINFECTION DES MATÉRIAUX À L'OZONE

(30) Priority: 12.05.2023 CZ 20230194
(43) Date of publication of application: 18.03.2026
(73) Proprietor: Ozone Cleaner Technology s.r.o., 709 00 Ostrava (CZ)
(72) Inventor: STRIZEK, Matej, 71600 Ostrava (CZ); TKACH, Mykola, 70300 Ostrava (CZ); DVORSKY, Richard, 70030 Ostrava (CZ); BERGER, Jan, 72300 Ostrava (CZ)
(74) Representative: Dadej, Leopold
(86) International application number: PCT/CZ2024/050033
(87) International publication number: WO 2024/235370

(56) References cited:
- JP-A- 2007 089 936

## Description

### Technical Field

The present invention relates both to a device for disinfection of material (for example, medical material such as respirators) with ozone and to a device for general disinfection of toxic waste, wherein it particularly relates to a device with a rotating drum for providing homogeneous, full-area disinfection of parts being disinfected.

### Background of the Invention

Especially in the medical environment, it is necessary to ensure that patients, professional staff or other persons are not exposed to various pathogens, or that the level of exposure to such pathogens, such as molds, is reduced as effectively as possible. Mold colonies, often invisible to the eye, also represent an accumulator of surface humidity, which provides a breeding ground for other pathogens and allergens, such as mite feces, spores, bacteria, viruses, amoebae, plant debris, etc. These harmful organisms can be found e.g. on sheets, bedding, or other fabric materials that patients or staff come into contact with, or on drapes or other medical material. This material must therefore be disinfected before repeated use.

The second, very serious problem is the destructive disposal of used material in the BIOHAZARD category, which does not assume its repeated use for its original purpose. The current costs for its safe disposal, e.g. by incineration, are relatively high. If the biological contamination of the material is removed by a preliminary disinfection, the cost of disposal of such non-toxic material is significantly reduced.

Currently, ozone is also used for the disinfection of material, which has strong oxidizing effects and, in combination with the appropriate humidity level, simultaneously creates highly reactive hydroxyl and super oxygen radicals. In this complex form, ozone is known for its strong antifungal, antibacterial, and virucidal properties. An advantage of ozone also lies in the fact that it can be produced relatively easily from air, e.g. by using an ozone generator that works on the principle of splitting the molecules of oxygen O₂ by corona discharge.

Devices that use ozone for disinfection are most often implemented in the form of various boxes or cabinets. An example of such a solution in the patent literature is, for example, the solution described in the Indian application IN202011020231 A, where the drapes are placed on a tray and inserted into a disinfection chamber in which ozone is generated. However, a disadvantage of such solutions lies in the fact that the individual objects being disinfected, or parts thereof, are exposed to the action of ozone with different exposure due to mutual contact and overlaps, resulting in significant inhomogeneity of disinfection. Thus, some parts of the material being disinfected may be insufficiently disinfected.

In order to improve the homogeneity of disinfection, solutions with a rotating drum in which the material being disinfected is placed are also known in the state of the art, wherein the rotation of the drum causes the material to tumble and mix. Such a device is disclosed e.g. in the patent EP3743118 A1. This document describes a disinfection device which, in addition to a disinfection chamber, also comprises an ozone generation chamber and an ozone reduction chamber. The resulting device is thus quite complex and space-consuming. In addition, a sufficiently intensive ozone circulation through the material being disinfected inside the rotating drum is not ensured. While the drum may be perforated to allow ozone to pass into the drum, these openings must be used as both an ozone inlet into the drum and ozone outlet out of the drum, which may result in unsatisfactory ozone circulation. The application is mainly focused on the air/ozone circulation between the disinfection chamber, the ozone generation chamber and the ozone reduction chamber. Another disinfection device is known from JP 2007 089936 A.

It would therefore be desirable to come up with a disinfection device solution that would be characterized by a relatively simple and space-saving arrangement and that would at the same time allow reliable and intensive circulation of ozone through the material being disinfected inside the rotating drum to ensure better homogeneity of disinfection.

### Summary of the Invention

The above shortcomings are to a large extent eliminated by a device for disinfection of material with ozone comprising a hermetically sealed working chamber, at least one ozone generator arranged in the working chamber, and an outer drum arranged in the working chamber and rotationally movable relative to the working chamber. The essence of the device of the present invention lies in the fact that the device further comprises an inner drum arranged at least partially inside the outer drum, wherein the outer drum and the inner drum have a cylindrical shape, wherein between the inner wall of the outer drum and the outer wall of the inner drum, an inner disinfection space for placing material being disinfected is defined, and between the inner wall of the working chamber and the outer wall of the outer drum, an outer space of the working chamber is defined. The inner drum comprises at least one inlet opening of the inner drum connecting the outer space of the working chamber to the inner space of the inner drum and at least one outlet opening of the inner drum arranged in a lateral surface of the inner drum and connecting the inner space of the inner drum to the inner disinfection space, and the outer drum comprises at least one outlet opening of the outer drum arranged in a lateral surface of the outer drum and connecting the inner disinfection space to the outer space of the working chamber. The device further comprises at least one fan adapted to blow air through at least one inlet opening of the inner drum into the inner space of the inner drum.

An advantage of the device of the present invention lies primarily in the fact that it allows reliable and intensive circulation of ozone through the material being disinfected, which, in combination with the placement of the material being disinfected in the rotating drum, provides excellent homogeneity of disinfection. In addition, the arrangement of the device is relatively simple and space-saving and can be thus used in a variety of applications - from hospital environments to home use. Moreover, the device can also be used for disinfection of biohazard category waste prior to its normal disposal. If the biological contamination of the material is removed by a preliminary disinfection, the cost of disposal of such non-toxic material is significantly reduced.

At least one ozone generator is preferably arranged in the outer space of the working chamber. Therefore, in this embodiment, ozone formation occurs outside the outer drum. The outer space of the working chamber may be spacious enough to accommodate a larger number of ozone generators for a stronger disinfecting effect.

For example, the at least one ozone generator is arranged in the inner space of the inner drum. Thus, in this embodiment, ozone is formed in the inner space of the inner drum, i.e. closer to the material being disinfected itself.

The at least one fan is preferably arranged in the region of the inlet opening of the inner drum. This ensures a reliable and efficient blowing of air from the outer space of the working chamber to the inner space of the inner drum.

The device preferably comprises two fans, wherein the inner drum comprises two mutually opposed inlet openings of the inner drum, wherein one fan is arranged in the region of each inlet opening. Such an arrangement with two fans ensures intensive circulation of ozone through the disinfection device. In addition, the air with ozone flows symmetrically through the device and enters the inner drum from both sides, which also contributes to a higher homogeneity of disinfection.

Preferably, the fan comprises an axial turbine which allows axial blowing of air into the inner space of the inner drum.

The inner drum is preferably rotationally movable relative to the working chamber. The rotating movement of the inner drum can also contribute to the homogeneity of the disinfection, but the rotating movement of the outer drum, in which the material being disinfected that is being tumbled in this outer drum due to the rotation is placed, is essential for the function of the device. The inner drum can rotate together with the outer drum, or the rotation of the inner drum can be implemented independently of the rotation of the outer drum such that both drums can rotate at different speeds, for example.

The rotational movement of the outer drum relative to the working chamber is preferably implemented by means of a circumferential drive of the outer drum, a shaft of the circumferential drive of the outer drum, and at least one pulley of the circumferential drive of the outer drum, wherein the shaft of the circumferential drive of the outer drum is connected to the pulley of the circumferential drive of the outer drum and the outer drum abuts on the pulley of the circumferential drive of the outer drum. The pulleys of the circumferential drive of the outer drum may be implemented with teeth on which the teeth of the outer drum abut, or they may not comprise such teeth and the rotating movement is transmitted only by friction.

The device further preferably comprises at least two guide pulleys and at least two shafts of the guide pulleys, wherein each shaft of the guide pulleys is connected to at least one guide pulley. The guide pulleys and the shafts of the guide pulleys are used to stabilize the rotational movement of the outer drum in the working chamber. For example, the device may comprise two shafts of the guide pulleys, wherein for example, two guide pulleys are fixed to each shaft of the guide pulleys. These two shafts of the guide pulleys, together with the shaft of the circumferential drive of the outer drum, are arranged in such a way that they are angularly offset by 120° relative to each other. Alternatively, the device may comprise, for example, three shafts of the guide pulleys that are, together with the shaft of the circumferential drive of the outer drum, arranged in such a way that they are angularly offset by 90° relative to each other.

At least one outlet opening of the inner drum and/or at least one outlet opening of the outer drum is preferably rounded on the side of the inner disinfection space. Most preferably, each outlet opening of the inner drum and each outlet opening of the outer drum is rounded in this way, which ensures that the material being disinfected slides smoothly in the disinfection space without catching on the edges of the respective openings.

### Description of Drawings

A summary of the invention is further clarified using exemplary embodiments thereof, which are described with reference to the accompanying drawings, in which:
fig. 1 schematically shows the device for disinfection of material in the first exemplary embodiment of the present invention,
fig. 2 schematically shows the device for disinfection of material in the first exemplary embodiment of the present invention, with a representation of the airflow direction,
fig. 3 schematically shows the device for disinfection of material in the second exemplary embodiment of the present invention, and
fig. 4 schematically shows the device for disinfection of material in the third exemplary embodiment of the present invention.

### Exemplary Embodiments of the Invention

The invention will be further clarified using exemplary embodiments with reference to the respective drawings.

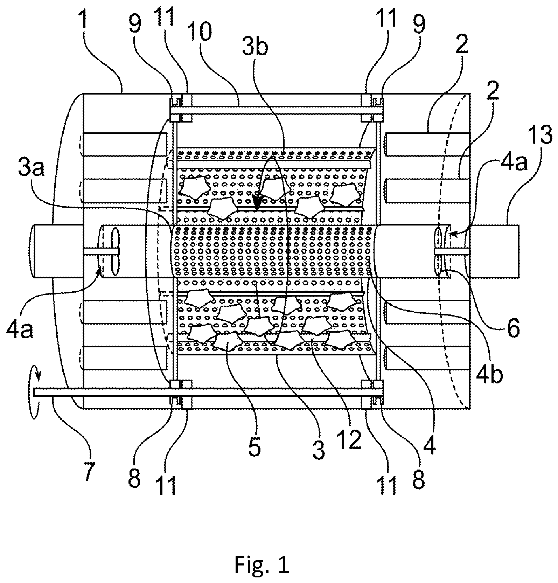

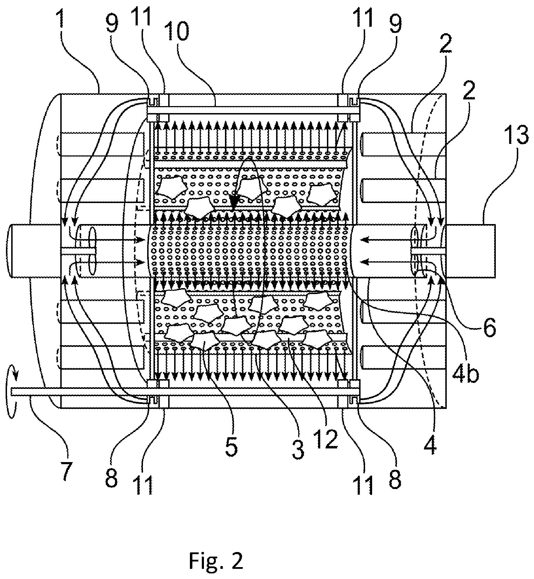

In the first exemplary embodiment of fig. 1 and fig. 2, the device for disinfection of material comprises a hermetically sealed working chamber 1, at least one ozone generator 2, an outer drum 3, an inner drum 4, and at least one fan 6 for blowing air into the inner drum 4. The outer drum 3 is arranged in the working chamber 1 and is rotationally movable relative to the working chamber 1.

The rotational movement of the outer drum 3 is implemented for example by the drive of the outer drum 3, wherein the movement of the drive of the outer drum 3 is converted to the rotational movement of the outer drum 3 by a shaft 7 of the circumferential drive of the outer drum 3. The shaft 7 of the circumferential drive of the outer drum 3 is connected to the outer drum 3 via a pulley 8 of the circumferential drive of the outer drum 3, in particular via two pulleys 8 of the circumferential drive of the outer drum 3. As an example, these pulleys 8 of the circumferential drive of the outer drum 3 have teeth, wherein the outer drum 3 abuts on the toothed pulley 8 of the circumferential drive of the outer drum 3 by its toothed circumference. Alternatively, the pulleys 8 of the circumferential drive of the outer drum 3 do not need to have teeth and the rotating movement is provided by friction.

The rotational movement of the outer drum 3 relative to the working chamber 1 is further stabilized by guide pulleys 9, which are placed on the shaft 10 of the guide pulleys 9. A bearing housing 11 is placed on the shaft 10 of the guide pulleys 9 at the respective guide pulley 9, wherein the bearing housings 11 are also placed on the shaft 7 of the circumferential drive of the outer drum 3 at the pulley 8 of the circumferential drive of the outer drum 3. All the bearing housings 11 are fixed to the inner wall of the working chamber 1. As an example, 3 to 6 guide pulleys 9 are used, however, alternatively a different number of the guide pulleys 9 can also be used. For example, the device may comprise two shafts 10 of the guide pulleys 9, wherein for example two guide pulleys 9 are fixed to each shaft 10 of the guide pulleys 9. These two shafts 10 of the guide pulleys 9, together with the shaft 7 of the circumferential drive of the outer drum 3, are arranged in such a way that they are angularly offset by 120° relative to each other. Alternatively, the device may comprise, for example, three shafts 10 of the guide pulleys 9 that are, together with the shaft 7 of the circumferential drive of the outer drum 3, arranged in such a way that they are angularly offset by 90° relative to each other.

Alternatively, the rotation of the outer drum 3 relative to the working chamber 1 can also be implemented in other ways, for example, the rotational movement of the shaft 7 of the circumferential drive of the outer drum 3 (this movement is in fig. 1 or fig. 2 indicated by an arrow) can be converted into the rotation of the outer drum 3 by means of other elements. The rotational movement of the outer drum 3 is in fig. 1 or fig. 2 also indicated by an arrow, wherein, for example, the outer drum 3 rotates at a speed of 30 to 120 revolutions per minute.

The inner drum 4 is arranged at least partially inside the outer drum 3, wherein in the first exemplary embodiment this inner drum 4 also rotates relative to the working chamber 1. Specifically, the rotational movement of the inner drum 4 is implemented by the same mechanism as that of the outer drum 3, i.e., for example, by means of the shaft 7 of the circumferential drive of the outer drum 3, wherein the inner drum 4 moves together with the outer drum 3 due to the fact that the inner drum 4 is firmly connected to the outer drum 3. The inner drum 4 and the outer drum 3 together form the drum chamber. Alternatively, the rotational movement of the inner drum 4 may be implemented independently of the rotation of the outer drum 3, e.g. by means of its own drive, and therefore it does not have to be firmly connected to the outer drum 3 but can be only placed in the working chamber 1. Alternatively, the inner drum 4 does not have to be rotational. The rotational movement of the outer drum 3 is crucial to ensure the homogeneity of disinfection, since it is in the outer drum 3, specifically in the space between the walls of the outer drum 3 and the inner drum 4, that the material 5 being disinfected is placed. As a result of the rotation of the outer drum 3, the material 5 being disinfected is tumbled in the said space, wherein to prevent the material 5 being disinfected from sliding, the inner side is provided with blades 12. The number, shape, or dimensions of these blades 12 may vary, wherein, for example, the blades 12 have a flat shape of a length of 40 mm to 200 mm, and 4 to 16 blades 12 are arranged at a mutual distance on the inner side of the outer drum 3.

The outer drum 3 and the inner drum 4 have a cylindrical shape, wherein the inner drum 4 is arranged concentrically in the outer drum 3, i.e. the axial axes of the outer drum 3 and the inner drum 4 coincide. As can be seen e.g. in fig. 1 or fig. 2, the inner drum 4 protrudes from the outer drum 3 at the sides (namely, at the bases of the outer drum 3). The inner drum 4 is thus arranged inside the outer drum 3, however, it does not have to be arranged in the outer drum 3 in its entirety. It is therefore arranged therein at least partially.

Both the outer drum 3 and the inner drum 4 are perforated and therefore allow air to pass through, wherein the airflow is provided by at least one fan 6. For greater clarity of the device of the present invention, the individual spaces and the openings connecting these spaces will be defined.

The outer space of the working chamber 1 and the inner disinfection space will be defined first. The outer space of the working chamber 1 is defined between the walls of the outer drum 3 and the working chamber 1, namely between the outer wall of the outer drum 3 and the inner wall of the working chamber 1. It is therefore the space inside the working chamber 1 but outside the outer drum 3. The inner disinfection space is defined between the walls of the outer drum 3 and the inner drum 4, particularly between the inner wall of the outer drum 3 and the outer wall of the inner drum 4. It is therefore the space inside the outer drum 3 but outside the inner drum 4, i.e. the space that is located between the lateral surfaces of the outer drum 3 and inner drum 4. In the first exemplary embodiment of fig. 1 or fig. 2, at least one ozone generator 2 is arranged in the outer space of the working chamber 1, wherein the material 5 being disinfected is placed in the inner disinfection space.

The inner drum 4 comprises at least one inlet opening 4a of the inner drum 4 and at least one outlet opening 4b of the inner drum 4. The inlet opening 4a of the inner drum 4 connects the outer space of the working chamber 1 to the inner space of the inner drum 4, and thus allows airflow from the outer space of the working chamber 1 to the inner space of the inner drum 4. In the first exemplary embodiment of fig. 1 or fig. 2, the inner drum 4 comprises two inlet openings 4a of the inner drum 4, which are arranged in two bases of the inner drum 4. Therefore, the air, or rather a mixture of air and ozone, flows into the inner space of the inner drum 4 from both sides, as will be described in more detail below.

In the first exemplary embodiment, the inner drum 4 comprises a number of outlet openings 4b of the inner drum 4 that connect the inner space of the inner drum 4 to the inner disinfection space, and thus allow air (or a mixture of air and ozone) to flow from the inner space of the inner drum 4 to the inner disinfection space. A number of outlet openings 4b of the inner drum 4 is arranged in the lateral surface of the inner drum 4, wherein the size and axial distribution of the outlet openings 4b of the inner drum is selected so as to achieve an aerodynamically uniform outlet of air along the entire length of the inner drum 4 into the inner disinfection space. The outlet openings 4b of the inner drum 4 may also be rounded on the side of the inner disinfection space. The diameter of the outlet openings 4b of the inner drum 4 varies, for example, from 5 mm to 50 mm.

In the first exemplary embodiment, the outer drum 3 comprises a number of outlet openings 3b of the outer drum 3 that connect the inner disinfection space to the outer space of the working chamber 1 and thus allow airflow from the inner disinfection space to the outer space of the working chamber 1. The number of the outlet openings 3b of the outer drum 3 is arranged in the lateral surface of the outer drum 3, wherein the outlet openings 3b of the outer drum 3 may also be rounded on the side of the inner disinfection space. Such rounding ensures smooth sliding of the material 5 being disinfected without catching on the edges of the openings. The diameter of the outlet openings 3b of the outer drum 3 varies, for example, from 5 mm to 50 mm. Fig. 1 also indicates an inlet opening 3a of the outer drum 3, however, essentially, the air flows into the outer drum 3 via the inner drum 4. In the embodiment of fig. 1, these inlet openings 3a of the outer drum 3 thus correspond to the openings in the individual bases of the outer drum 3 into which the inner drum 4 is inserted and fixed during the manufacture. However, the function of the inlet openings 3a of the outer drum 3 is also fulfilled by the outlet openings 4b of the inner drum 4.

The device for disinfection of material with ozone also comprises at least one fan 6 adapted to blow air into the inner space of the inner drum 4, wherein the fan 6 provides air circulation in the hermetically sealed working chamber 1 as shown by the arrows in fig. 2. The fan 6 is preferably arranged in the region of the inlet opening 4a of the inner drum 4 and is driven by the drive 13 of the fan 6. For example, the fan 6 is implemented as an axial turbine and the drive 13 of the fan 6 is implemented as an axial turbine engine. The axis of rotation of the axial turbine preferably coincides with the axis of rotation of the outer drum 3 and the inner drum 4.

The hermetically sealed working chamber 1 can be operated at standard, i.e. atmospheric, air pressure, or alternatively it can be evacuated to negative pressure (e.g. up to -0.5 bar) before use and supplemented with oxygen and water vapor to the required concentration. Thus, oxygen and water vapor feed can be connected to the device, wherein the mentioned enrichment leads to a higher efficiency in both ozone production by the ozone generator 2 and in the production of highly reactive hydroxyl and super oxygen radicals, and thus also to a higher efficiency of the disinfection process. In the working chamber 1, the increased relative humidity is provided to ensure effective action of hydroxyl and superoxide radicals.

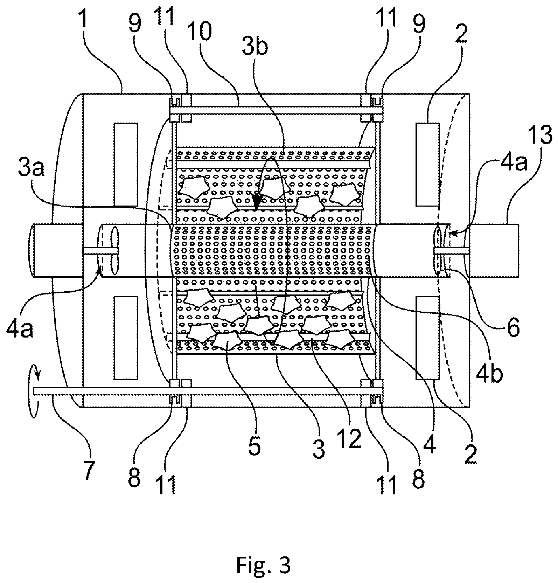

In the first exemplary embodiment of fig. 1 or fig. 2, the at least one ozone generator 2 is arranged in the outer disinfection space. The ozone generators 2 may use e.g. corona discharge to produce ozone and may be arranged in the working chamber 1 e.g. axially, in varying numbers. In an alternative embodiment of fig. 3, the ozone generators 2 may be arranged radially in the outer disinfection space.

Thus, in the first and second exemplary embodiments, ozone is created in the outer space of the working chamber 1, where it is produced by the ozone generators 2. Subsequently, the ozone-containing air or the oxygen-enriched and ozone-containing air is blown through the inlet openings 4a of the inner drum 4 into the inner space of the inner drum 4 by means of the fans 6, as shown by the arrows in fig. 2. As can be further seen in fig. 2, the outlet openings 4b of the inner drum 4 are arranged only in that part of that inner drum 4 that is located inside the outer drum 3. On the other hand, the parts of the inner drum 4 that protrude from the outer drum 3, are implemented as solid, without openings. This allows reliable blowing of air from the outer space of the working chamber 1 to the inner space of the inner drum 4. From there, the air flows through the outlet openings 4b of the inner drum 4 into the inner disinfection space, where it acts on the material 5 being disinfected, which is tumbled due to the rotation of the outer drum 3 in the inner disinfection space. As also indicated by the arrows, the air then exits back into the outer space of the working chamber 1, where it is re-ozonized by means of the ozone generators 2, and is then again sucked radially by means of the fans 6 and blown into the inner space of the inner drum 4. In this way, the air circulates through the device of the present invention, and the material 5 being disinfected is continuously disinfected with ozone. To safely complete the disinfection process, ozone molecules need to be inactivated by conductive carbon filters on the outlet pipe.

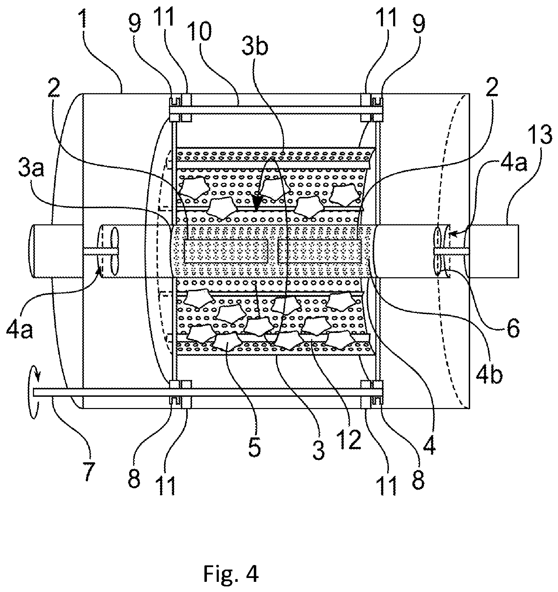

In the third exemplary embodiment of fig. 4, the at least one ozone generator 2 is arranged in the inner space of the inner drum 4, wherein the air circulation by means of the fans 6 is essentially the same as in the first and second exemplary embodiments, with the difference that the ozone is created in the inner space of the inner drum 4. Alternatively, the ozone generators 2 may be arranged in the inner space of the inner drum 4 as well as in the outer space of the working chamber 1.

### Industrial applicability

The device described above can be used for non-destructive disinfection of various materials, not only in the medical field but also e.g. in households, and on an industrial scale for disinfection of biohazard category waste prior to its normal disposal.

### List of reference signs

- 1 -: working chamber
- 2 -: ozone generator
- 3 -: outer drum
- 3a -: inlet opening of the outer drum
- 3b -: outlet opening of the outer drum
- 4 -: inner drum
- 4a -: inlet opening of the inner drum
- 4b -: outlet opening of the inner drum
- 5 -: material being disinfected
- 6 -: fan
- 7 -: shaft of the circumferential drive of the outer drum
- 8 -: pulley of the circumferential drive of the outer drum
- 9 -: guide pulley
- 10 -: shaft of the guide pulleys
- 11 -: bearing housing
- 12 -: blade
- 13 -: drive of the fan

## Claims

1. A device for disinfection of material with ozone comprising a hermetically sealed working chamber (1), at least one ozone generator (2) arranged in the working chamber (1), and an outer drum (3) arranged in the working chamber (1) and rotationally movable relative to the working chamber (1), wherein the device further comprises an inner drum (4) arranged at least partially inside the outer drum (3), wherein the outer drum (3) and the inner drum (4) have a cylindrical shape, wherein between the inner wall of the outer drum (3) and the outer wall of the inner drum (4) an inner disinfection space for placing material (5) being disinfected is defined, and between the inner wall of the working chamber (1) and the outer wall of the outer drum (3) an outer space of the working chamber (1) is defined, wherein the inner drum (4) comprises at least one inlet opening (4a) of the inner drum (4) connecting the outer space of the working chamber (1) to the inner space of the inner drum (4) and at least one outlet opening (4b) of the inner drum (4) arranged in a lateral surface of the inner drum (4) and connecting the inner space of the inner drum (4) to the inner disinfection space, **characterised in that** the outer drum (3) comprises at least one outlet opening (3b) of the outer drum (3) arranged in a lateral surface of the outer drum (3) and connecting the inner disinfection space to the outer space of the working chamber (1), wherein the device further comprises at least one fan (6) adapted to blow air through at least one inlet opening (4a) of the inner drum (4) into the inner space of the inner drum (4).

2. The device according to claim 1, wherein at least one ozone generator (2) is arranged in the outer space of the working chamber (1).

3. The device according to any one of the preceding claims 1 and 2, wherein at least one ozone generator (2) is arranged in the inner space of the inner drum (4).

4. The device according to any one of the preceding claims 1 to 3, wherein at least one fan (6) is arranged in the region of the inlet opening (4a) of the inner drum (4).

5. The device according to any one of the preceding claims 1 to 4, wherein it comprises two fans (6), wherein the inner drum (4) comprises two mutually opposed inlet openings (4a) of the inner drum (4), wherein one fan (6) is arranged in the region of each inlet opening (4a).

6. The device according to any one of the preceding claims 1 to 5, wherein the fan (6) comprises an axial turbine.

7. The device according to any one of the preceding claims 1 to 6, wherein the inner drum (4) is rotationally movable relative to the working chamber (1).

8. The device according to any one of the preceding claims 1 to 7, wherein the rotational movement of the outer drum (3) relative to the working chamber (1) is implemented by means of a circumferential drive of the outer drum (3), a shaft (7) of the circumferential drive of the outer drum and at least one pulley (8) of the circumferential drive of the outer drum (3), wherein the shaft (7) of the circumferential drive of the outer drum (3) is connected to the pulley (8) of the circumferential drive of the outer drum (3) and the outer drum (3) abuts on the pulley (8) of the circumferential drive of the outer drum (3).

9. The device according to claim 8, wherein it further comprises at least two guide pulleys (9) and at least two shafts (10) of the guide pulleys (9), wherein each shaft (10) of the guide pulleys (9) is connected to at least one guide pulley (9).

10. The device according to any one of the preceding claims 1 to 9, wherein at least one outlet opening (4b) of the inner drum (4) and/or at least one outlet opening (3b) of the outer drum (3) is rounded on the side of the inner disinfection space.

## Patentansprüche

1. Eine Vorrichtung zur Desinfektion von Material mit Ozon, umfassend eine hermetisch abgeschlossene Arbeitskammer (1), mindestens einen in der Arbeitskammer (1) angeordneten Ozongenerator (2), und eine in der Arbeitskammer (1) angeordnete und relativ zur Arbeitskammer (1) drehbare Außentrommel (3), wobei die Vorrichtung ferner eine zumindest teilweise innerhalb der Außentrommel (3) angeordnete Innentrommel (4) umfasst, wobei die Außentrommel (3) und die Innentrommel (4) eine zylindrische Form aufweisen, wobei zwischen der Innenwand der Außentrommel (3) und der Außenwand der Innentrommel (4) ein innerer Desinfektionsraum zum Einbringen von zu desinfizierendem Material (5) definiert ist und zwischen der Innenwand der Arbeitskammer (1) und der Außenwand der Außentrommel (3) ein Außenraum der Arbeitskammer (1) definiert ist, wobei die Innentrommel (4) mindestens eine Einlassöffnung (4a) der Innentrommel (4) aufweist, die den Außenraum der Arbeitskammer (1) mit dem Innenraum der Innentrommel (4) verbindet, sowie mindestens eine in einer Seitenfläche der Innentrommel (4) angeordnete Auslassöffnung (4b) der Innentrommel (4), die den Innenraum der Innentrommel (4) mit dem inneren Desinfektionsraum verbindet, **dadurch gekennzeichnet, dass** die Außentrommel (3) mindestens eine Auslassöffnung (3b) der Außentrommel (3) aufweist, die in einer Seitenfläche der Außentrommel (3) angeordnet ist und den inneren Desinfektionsraum mit dem Außenraum der Arbeitskammer (1) verbindet, wobei die Vorrichtung ferner mindestens einen Ventilator (6) umfasst, der dazu ausgelegt ist, Luft durch mindestens eine Einlassöffnung (4a) der Innentrommel (4) in den Innenraum der Innentrommel (4) zu blasen.

2. Die Vorrichtung nach Anspruch 1, wobei mindestens ein Ozongenerator (2) im Außenraum der Arbeitskammer (1) angeordnet ist.

3. Die Vorrichtung nach einem der vorstehenden Ansprüche 1 und 2, wobei mindestens ein Ozongenerator (2) im Innenraum der Innentrommel (4) angeordnet ist.

4. Die Vorrichtung nach einem der vorstehenden Ansprüche 1 bis 3, wobei mindestens ein Ventilator (6) im Bereich der Einlassöffnung (4a) der Innentrommel (4) angeordnet ist.

5. Die Vorrichtung nach einem der vorstehenden Ansprüche 1 bis 4, wobei sie zwei Ventilatoren (6) umfasst, wobei die Innentrommel (4) zwei einander gegenüberliegende Einlassöffnungen (4a) der Innentrommel (4) aufweist, wobei im Bereich jeder Einlassöffnung (4a) ein Ventilator (6) angeordnet ist.

6. Die Vorrichtung nach einem der vorstehenden Ansprüche 1 bis 5, der Ventilator (6) eine Axialturbine umfasst.

7. Die Vorrichtung nach einem der vorstehenden Ansprüche 1 bis 6, wobei die Innentrommel (4) relativ zur Arbeitskammer (1) drehbar ist.

8. Die Vorrichtung nach einem der vorstehenden Ansprüche 1 bis 7, wobei die Drehbewegung der Außentrommel (3) relativ zur Arbeitskammer (1) mittels eines Umfangsantriebs der Außentrommel (3), einer Welle (7) des Umfangsantriebs der Außentrommel und mindestens einer Riemenscheibe (8) des Umfangsantriebs der Außentrommel (3) realisiert wird, wobei die Welle (7) des Umfangsantriebs der Außentrommel (3) mit der Riemenscheibe (8) des Umfangsantriebs der Außentrommel (3) verbunden ist und die Außentrommel (3) an der Riemenscheibe (8) des Umfangsantriebs der Außentrommel (3) anliegt.

9. Die Vorrichtung nach Anspruch 8, wobei sie ferner mindestens zwei Führungsriemenscheiben (9) und mindestens zwei Wellen (10) der Führungsriemenscheiben (9) umfasst, wobei jede Welle (10) der Führungsriemenscheiben (9) mit mindestens einer Führungsriemenscheibe (9) verbunden ist.

10. Die Vorrichtung nach einem der vorstehenden Ansprüche 1 bis 9, wobei mindestens eine Auslassöffnung (4b) der Innentrommel (4) und/oder mindestens eine Auslassöffnung (3b) der Außentrommel (3) auf der Seite des inneren Desinfektionsraums abgerundet ist.

## Revendications

1. Un dispositif de désinfection de matériaux à l'ozone, comprenant une chambre de travail (1) hermétiquement fermée, au moins un générateur d'ozone (2) disposé dans la chambre de travail (1), et un tambour extérieur (3) disposé dans la chambre de travail (1) et pouvant tourner par rapport à la chambre de travail (1), où le dispositif comprend en outre un tambour intérieur (4) disposé au moins partiellement à l'intérieur du tambour extérieur (3), où le tambour extérieur (3) et le tambour intérieur (4) ont une forme cylindrique, où, entre la paroi intérieure du tambour extérieur (3) et la paroi extérieure du tambour intérieur (4) est défini un espace de désinfection intérieur destiné à recevoir le matériau (5) à désinfecter, et entre la paroi intérieure de la chambre de travail (1) et la paroi extérieure du tambour extérieur (3) est défini un espace extérieur de la chambre de travail (1), où le tambour intérieur (4) comprend au moins une ouverture d'entrée (4a) du tambour intérieur (4) reliant l'espace extérieur de la chambre de travail (1) à l'espace intérieur du tambour intérieur (4) et au moins une ouverture de sortie (4b) du tambour intérieur (4) disposée dans une surface latérale du tambour intérieur (4) et reliant l'espace intérieur du tambour intérieur (4) à l'espace de désinfection intérieur, **caractérisé en ce que** le tambour extérieur (3) comprend au moins une ouverture de sortie (3b) du tambour extérieur (3), disposée dans une surface latérale du tambour extérieur (3) et reliant l'espace de désinfection intérieur à l'espace extérieur de la chambre de travail (1), où le dispositif comprend en outre au moins un ventilateur (6) adapté pour souffler de l'air à travers au moins une ouverture d'entrée (4a) du tambour intérieur (4) dans l'espace intérieur du tambour intérieur (4).

2. Le dispositif selon la revendication 1, où au moins un générateur d'ozone (2) est disposé dans l'espace extérieur de la chambre de travail (1).

3. Le dispositif selon l'une quelconque des revendications 1 et 2 précédentes, où au moins un générateur d'ozone (2) est disposé dans l'espace intérieur du tambour intérieur (4).

4. Le dispositif selon l'une quelconque des revendications 1 à 3 précédentes, où au moins un ventilateur (6) est disposé dans la région de l'ouverture d'entrée (4a) du tambour intérieur (4).

5. Le dispositif selon l'une quelconque des revendications 1 à 4 précédentes, où il comprend deux ventilateurs (6), où le tambour intérieur (4) comprend deux ouvertures d'entrée (4a) du tambour intérieur (4) situées en vis-à-vis, où un ventilateur (6) est disposé dans la région de chaque ouverture d'entrée (4a).

6. Le dispositif selon l'une quelconque des revendications 1 à 5 précédentes, où le ventilateur (6) comprend une turbine axiale.

7. Le dispositif selon l'une quelconque des revendications 1 à 6 précédentes, où le tambour intérieur (4) est mobile en rotation par rapport à la chambre de travail (1).

8. Le dispositif selon l'une quelconque des revendications 1 à 7 précédentes, où le mouvement de rotation du tambour extérieur (3) par rapport à la chambre de travail (1) est mis en œuvre au moyen d'un entraînement circonférentiel du tambour extérieur (3), d'un arbre (7) de l'entraînement circonférentiel du tambour extérieur et d'au moins une poulie (8) de l'entraînement circonférentiel du tambour extérieur (3), où l'arbre (7) de l'entraînement circonférentiel du tambour extérieur (3) est relié à la poulie (8) de l'entraînement circonférentiel du tambour extérieur (3) et le tambour extérieur (3) vient en butée contre la poulie (8) de l'entraînement circonférentiel du tambour extérieur (3).

9. Le dispositif selon la revendication 8, où il comprend en outre au moins deux poulies de guidage (9) et au moins deux arbres (10) des poulies de guidage (9), où chaque arbre (10) des poulies de guidage (9) est relié à au moins une poulie de guidage (9).

10. Le dispositif selon l'une quelconque des revendications 1 à 9 précédentes, où au moins une ouverture de sortie (4b) du tambour intérieur (4) et/ou au moins une ouverture de sortie (3b) du tambour extérieur (3) est arrondie du côté de l'espace de désinfection intérieur.
